Europäisches Patentamt

European Patent Office

Office européen des brevets

(19)

(11) Numéro de publication: **0 188 927**
**B1**

(12) **FASCICULE DE BREVET EUROPEEN**

(45) Date de publication du fascicule du brevet:
26.07.89

(21) Numéro de dépôt: **85402219.1**

(22) Date de dépôt: **15.11.85**

(51) Int. Cl.⁴: **A61F 2/02**

(54) **Filtre perfectionné en particulier pour la retenue de caillots sanguins.**

(30) Priorité: **29.11.84 FR 8418198**

(43) Date de publication de la demande:
**30.07.86 Bulletin 86/31**

(45) Mention de la délivrance du brevet:
**26.07.89 Bulletin 89/30**

(84) Etats contractants désignés:
**AT BE CH DE GB IT LI LU NL SE**

(56) Documents cités:
**DE-A- 3 417 738**
**FR-A- 2 512 678**
**FR-A- 2 525 896**
**GB-A- 1 129 712**
**US-A- 3 334 629**
**US-A- 4 214 587**
**US-A- 4 425 908**

(73) Titulaire: **Société dite : L.G. MEDICAL S.A., Avenue des Temps Modernes, F-86360 CHASSENEUIL DU POITOU(FR)**

(72) Inventeur: **Metais, Joel, La Cottenciere Berthegon, F-86420 Monts sur Guesnes(FR)**

(74) Mandataire: **Lerner, François, 5, rue Jules Lefèbvre, F-75009 Paris(FR)**

ACTORUM AG

## Description

L'invention a pour objet un filtre perfectionné destiné à être placé sur le trajet sanguin, en particulier sur un trajet veineux, pour la retenue de caillots sanguins.

Des filtres de ce type sont connus, et sont par exemple décrits dans le brevet US-A-3 952 747.

De façon générale, ces filtres se présentent sous la forme d'un petit panier tronconique qui s'accroche à l'intérieur d'une veine en aval du trajet que l'on veut filtrer ; il s'agit en général de la veine cave arrivant au coeur. On peut ainsi arrêter, avant leur entrée dans le coeur, les éventuels caillots sanguins pouvant se former et risquant d'entraîner des embolies.

Une difficulté propre à ce genre d'intervention est de positionner correctement le filtre avec son axe sensiblement dans l'axe de la veine dans laquelle il va s'accrocher. Pour faciliter cet accrochage, les pattes du filtre sont équipées habituellement de crochets. De façon générale pour introduire le filtre dans la veine, on le pousse dans celle-ci au moyen d'un conduit qui traverse la veine et dont le diamètre est inférieur à celui de cette dernière. Lorsque le filtre arrive à l'extrémité du conduit, il est donc laché dans la veine et l'expansion de ses pattes formées en crochet assure l'ancrage du filtre. Il apparaît à l'évidence qu'un tel "lacher" est dans la pratique extrêmement difficile à contrôler, et il n'y a en fait guère de chance que le filtre en panier occupe à l'intérieur de la veine la position la plus favorable avec son axe sensiblement parallèle à l'axe de la veine.

L'invention a pour objet d'éviter cette difficulté.

A cet effet, un filtre conforme à l'invention, du type formé par des pattes élastiques déployées sensiblement suivant une corolle conique issues d'une tête ogivale, se caractérise en ce que au moins quelques pattes précitées sont pourvues, vers leurs extrémités libres, d'appendices orientés sensiblement parallèlement à la paroi sensiblement cylindrique engendrée par une ligne génératrice parallèle à l'axe de ladite corolle conique et décrivant comme ligne directrice le périmètre d'ouverture de ladite corolle dans une position normale d'utilisation.

De cette façon, lorsque le filtre est introduit dans la veine, lesdits appendices se plaquent contre les parois de la veine en obligeant le filtre à se placer avec son axe sensiblement confondu avec l'axe de la veine.

Selon une caractéristique de construction préférée de l'invention, le filtre comporte au moins trois pattes pourvues d'appendices précités et réparties angulairement autour de l'axe du filtre. De cette façon on obtient un bon centrage automatique du filtre mis en place dans la veine.

Selon une caractéristique d'un mode de réalisation préféré de l'invention, les pattes pourvues d'appendices sont plus courtes que celles qui en sont dépourvues. De cette façon, lorsque le filtre est introduit dans la veine, les pattes pourvues d'appendices viennent se plaquer les premières contre la paroi interne de la veine en centrant le filtre, avant que les autres pattes, plus longues ne soient déployées et ne viennent s'accrocher sur la paroi interne de la veine.

L'invention et sa mise en oeuvre apparaîtront plus clairement à l'aide de la description qui va suivre faite en référence aux dessins annexés dans lesquels :

- la figure 1 montre en perspective schématiquement un filtre classique fabriqué selon l'art antérieur,
- la figure 2 montre schématiquement le filtre mis en place à l'intérieur d'une veine, ce filtre s'étant bloqué de travers dans la veine et non parallèlement à son axe,
- la figure 3 montre, comme la figure 1, un filtre modifié conformément à l'invention,
- la figure 4 est une vue par dessus faite selon la flèche IV de la figure 3,
- la figure 5 montre, comme la figure 2, le filtre modifié de l'invention, mis en place dans une veine et dont l'axe coïncide sensiblement avec celui de la veine,
- la figure 6 montre une vue de dessus de la figure 5, faite sensiblement selon la flèche VI de cette figure,
- les figures 7, 8 et 9 montrent trois étapes successives de la mise en place du filtre de l'invention dans une veine, selon les plans respectifs VII-VII et IX-IX de la figure 6,
- la figure 10 montre, à plus grande échelle, un détail de montage d'un appendice en bout d'une patte d'un filtre,
- les figures 11 et 12 montrent un autre détail de montage d'un appendice au bout d'une patte d'un filtre dans le cas où la patte est faite d'un fil métallique circulaire, la figure 11 montrant une vue en coupe faite sensiblement au niveau du plan XI-XI de la figure 12.

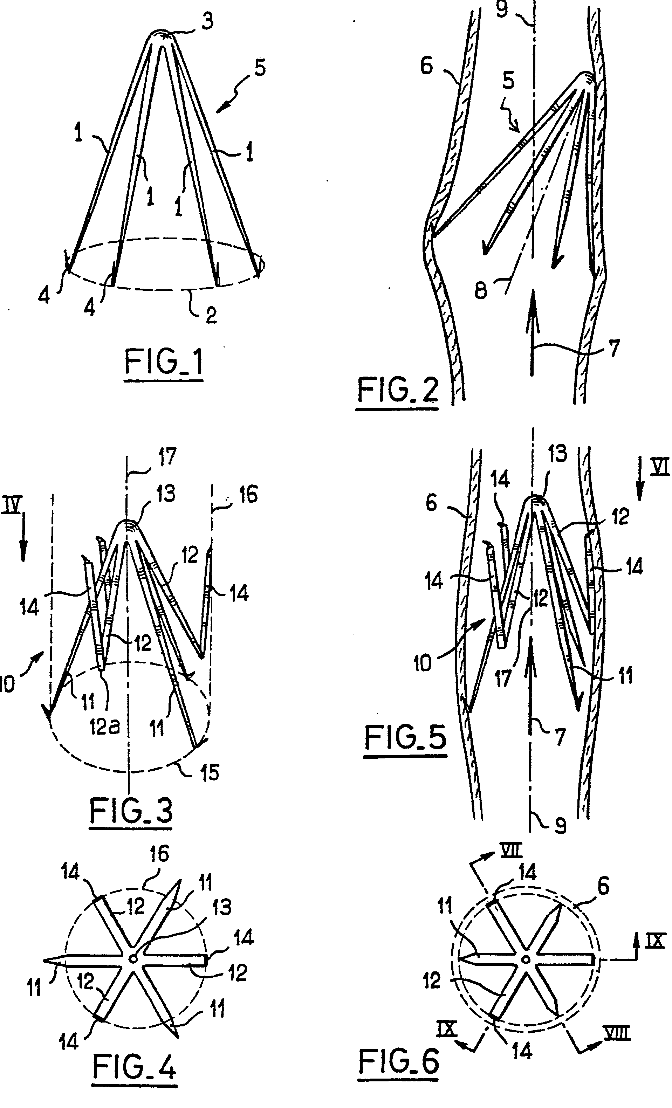

En se référant, tout d'abord aux figures 1 et 2, un filtre conforme à l'art antérieur est essentiellement constitué par des pattes 1, par exemple au nombre de quatre déployées sensiblement suivant une corolle conique dont l'ouverture a été marquée en pointillés en 2, ses pattes étant issues d'une tête ogivale commune 3. Les pattes sont terminées à leurs extrémités libres par des crochets 4.

La mise en place d'un tel filtre, à l'intérieur d'une veine, est connue et pratiquée couramment.

A la figure 2 on aperçoit le filtre 5 qui a été placé à l'intérieur d'une veine 6, le sens de l'écoulement sanguin étant indiqué par la flèche 7. Le filtre, comme cela se produit souvent se trouve placé avec son axe 8 nettement de travers par rapport à l'axe 9 de la veine, ce qui ne permet pas au filtre de travailler dans les meilleures conditions.

En se reportant aux figures 4 à 6, on voit qu'un filtre modifié conformément à l'invention, repéré dans son ensemble par le repère 10 comporte deux types de pattes différents. Dans l'exemple illustré le filtre comporte trois pattes 11 qui peuvent être constituées de manière identique aux pattes 1 du filtre 5 des figures 1 et 2, et trois pattes 12 plus courtes, les pattes 11 et 12 étant issues d'une tête ogivale 13. Comme il apparaît clairement aux figures 3 et 5, les pattes courtes 12 sont pourvues, vers leurs extrémités libres d'appendices 14 revenant sensiblement en arrière vers la pointe de l'ogive 13. En d'autres termes, les appendices 14 sont dirigés à partir des extrémités 12a des pattes 12 vers le côté de fermeture du cône que forme le filtre.

De façon plus précise, si l'on désigne par 15 le périmètre d'ouverture de la corolle que forme le filtre, et si l'on désigne par 16 le cylindre engendré par une ligne génératrice parallèle à l'axe 17 du cône formé par le filtre 10 et se déplaçant en décrivant la ligne 15, les appendices 14 sont dirigés de manière à être sensiblement parallèles à la paroi du cylindre 16. En d'autres termes, lorsque le filtre est en place dans la veine 6, comme illustré à la figure 5, les appendices 14 vont se plaquer contre la paroi interne de la veine 6 en centrant ainsi automatiquement l'axe 17 du filtre 10 sur l'axe 9 de la veine 6. En effet, le cylindre 16 et la veine 6 coïncident sensiblement, la courbe 15 ayant été définie comme le périmètre de la corolle d'ouverture du filtre dans sa position normale d'utilisation.

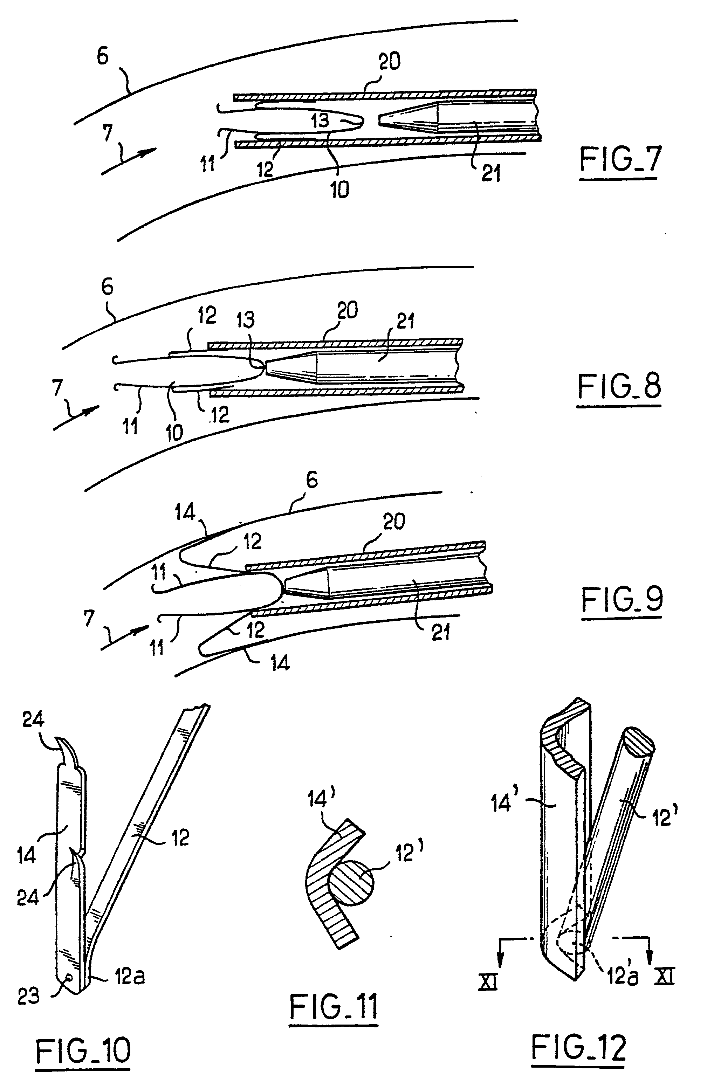

L'intérêt de choisir des pattes 12 un peu plus courtes que les pattes 11 apparaîtra maintenant à l'aide de la description des figures 7 à 9 expliquant la mise en place du filtre. A ces figures, on retrouve la veine 6 parcourue par le courant sanguin dans le sens de la flèche 7.

La mise en place du filtre est faite à travers un tube d'introduction repéré 20 désigné dans la technique "Desilet". Le filtre 10 est poussé à l'intérieur du tube 20 par un poussoir 21.

A la figure 7, le filtre apparaît avec ses pattes 11 sortant déjà du tube 20. On remarque que le tube 20 n'est pas parallèle à l'axe de la veine, comme cela arrive fréquemment. La figure 8 indique, un instant plus tard où le filtre est davantage sorti, les pattes 12 courtes ayant encore leurs extrémités arrière emprisonnées à l'intérieur du tube 20, et donc resserrées.

Il apparaît que sans les moyens de l'invention, un filtre lâché dans de telles conditions aurait toute chance de s'accrocher dans la veine de travers comme illustré à la figure 2.

A la figure 9 on aperçoit le filtre un instant plus tard avec les pattes 12 qui ont été libérées et qui se sont écartées, de sorte que les appendices 14 sont venus se bloquer en butée à l'intérieur de la paroi de la veine 6. Les pattes 11 plus longues sont encore emprisonnées dans le tube 20. Dans ces conditions, le filtre, sous l'action des pattes élastiques 12 qui s'arqueboutent sur leurs appendices 14, se place automatiquement sensiblement dans l'axe de la veine.

Un instant plus tard les pattes 11 seront libérées du tube 20 qui pourra être retiré, et l'on se trouvera dans la position illustrée aux figures 5 et 6.

En se reportant à la figure 10, on a illustré, à plus grande échelle, comment peut être constitué un appendice 14 et comment il peut être fixé vers l'extrémité libre 12a d'une patte 12, par exemple par un point de soudure 23. Si la nature du matériau le permet, l'appendice 14 peut être formé par simple repliage en arrière de l'extrémité d'une patte 12 plus longue, et par exemple de même longueur que les pattes 11.

Un ou plusieurs petits crochets 24 peuvent être prévus sur l'appendice 14 pour faciliter le blocage du filtre et assurer une action plus positive de ces pattes 12 pour centrage.

Il y a lieu de noter que les appendices 14 peuvent également favoriser un bon accrochage du filtre dans la veine et une meilleure tolérance, en permettant notamment de réduire l'agressivité des crochets d'ancrage, du fait de la plus grande surface d'ancrage que ces appendices présentent.

Selon les figures 11 et 12, on a illustré une conformation particulière d'appendice 14' dans le cas où les pattes 12' sont formées par un fil métallique rond. Dans ce cas, l'appendice 14' est profilé en cuvette ou V ouvert et la liaison peut être réalisée, par exemple, par soudure électrique vers l'extrémité 12'a libre du fil 12'.

Bien entendu l'invention n'est nullement limitée aux modes de réalisation illustrés décrits, le nombre des pattes pouvant varier, aussi bien pour les pattes pourvues d'appendices que pour celles qui en sont dépourvues, de même que leur forme et nature.

De manière générale, les pattes 12 pourvues d'appendices 14 peuvent être de longueur inférieure, égale ou supérieure aux pattes 11 qui en sont dépourvues. Cependant, les extrémités des appendices 14 seront en retrait par rapport à la tête 13 du filtre de façon que ces appendices soient éjectés du tube de mise en place avant la libération totale du filtre de sorte que les pattes 12 pourvues des appendices 14 seront les premières à prendre appui sur la paroi intérieure de la veine 6.

## Revendications

1. - Filtre destiné à être placé sur le trajet sanguin, en particulier sur un trajet veineux pour la retenue de caillots sanguins, du type formé par des pattes (11, 12) élastiques déployées sensiblement suivant une corolle conique issues d'une tête ogivale (13), caractérisé en ce que au moins quelques pattes (12) précitées, sont pourvues, vers leurs extrémités libres d'appendices (14) orientés sensiblement parallèlement à la paroi sensiblement cylindrique (16) engendrée par une ligne génératrice parallèle à l'axe (17) de ladite corolle conique et décrivant comme ligne directrice le périmètre d'ouverture (15) de ladite corolle dans une position normale d'utilisation.

2. - Filtre selon la revendication 1, caractérisé en ce que lesdits appendices (14) s'étendent au moins en partie, à partir des extrémités libres desdites pattes (12) qui en sont pourvues en revenant sensiblement en arrière et en étant dirigés vers le côté de fermeture du cône.

3. - Filtre selon la revendication 1 ou la revendication 2, caractérisé en ce qu'il comporte au moins trois pattes (12) pourvues d'appendices (14) précités et réparties angulairement autour de l'axe (17) précité du filtre.

4. - Filtre selon l'une des revendications précédentes, caractérisé en ce qu'il comporte au moins trois pattes (11) dépourvues d'appendices (14) précités et réparties angulairement autour de l'axe (17) précité du filtre.

5. - Filtre selon l'une des revendications précédentes, caractérisé en ce que lesdits appendices (14) sont pourvus de moyens d'accrochage (24) à la veine.

6. - Filtre selon l'une des revendications précédentes, caractérisé en ce que lesdits appendices (14) sont formés par repliage convenable des extrémités des pattes correspondantes (12).

7. - Filtre selon l'une des revendications 1 à 5, caractérisé en ce que lesdits appendices (14) sont rapportés sur des pattes (12) précitées.

8. - Filtre selon l'une des revendications précédentes, caractérisé en ce que les pattes (12) pourvues d'appendices (14) sont plus courtes que celles (11) qui en sont dépourvues.

9. - Filtre selon l'une des revendications 1 à 7 , caractérisé en ce que les pattes pourvues d'appendices sont plus longues que celles qui en sont dépourvues.

10. - Filtre selon l'une des revendications 1 à 7 caractérisé en ce que les pattes pourvues d'appendices sont de la même longueur que celles qui en sont dépourvues.

## Patentansprüche

1. In die Blutbahn, insbesondere in eine Venenbahn einsetzbarer Filter zum Zurückhalten von Blutgerinnseln, mit einem durch elastische Klauen, Pratzen oder Klammern (11, 12) gebildeten Aufbau, die sich im wesentlichen gemäß einer konischen Krone von einem spitzbogenförmigen Kopf (13) ausgehend erstrecken, dadurch gekennzeichnet, daß mindestens einige der Klauen (12) zu ihren freien Enden hin mit Fortsätzen (14) versehen sind, die im wesentlichen parallel zu dem im wesentlichen zylindrischen Mantel (16) ausgerichtet sind, der durch eine zur Achse (17) der konischen Krone parallele Erzeugende gebildet ist und als Leitkurve den Umfang der Öffnung (15) der Krone in einer normalen Verwendungsposition beschreibt.

2. Filter nach Anspruch 1, dadurch gekennzeichnet, daß die Ansätze (14) sich zumindest teilweise, ausgehend von den freien Enden der mit ihnen versehen Klauen (12) so erstrecken, daß sie merklich nach hinten verlaufen und gegen die Spitze oder Verschlußseite des Konus gerichtet sind.

3. Filter nach Anspruch 1 oder Anspruch 2, dadurch gekennzeichnet, daß er mindestens drei mit Ansätzen (14) versehene Klauen (12) umfaßt, die in Winkelverteilung um die Achse (17) des Filters herum angeordnet sind.

4. Filter nach einem der vorhergehenden Ansprüche, dadurch gekennzeichnet, daß er mindestens drei Klauen (11) ohne Ansätze (14) umfaßt, wobei diese Klauen in Winkelverteilung um die Achse (17) des Filters herumangeordnet sind.

5. Filter nach einem der vorhergehenden Ansprüche, dadurch gekennzeichnet, daß die Ansätze (14) mit Mitteln zum Verhaken (24) an der Vene versehen sind.

6. Filter nach einem der vorhergehenden Ansprüche, dadurch gekennzeichnet, daß die Ansätze durch geeignetes Biegen der entsprechenden Klauen (12) gebildet sind.

7. Filter nach einem der Ansprüche 1 bis 5, dadurch gekennzeichnet, daß die Ansätze (14) auf die Klauen (12) aufgestreckt sind.

8. Filter nach einem der vorhergehenden Ansprüche, dadurch gekennzeichnet, daß die mit Ansätzen (14) versehenen Klauen (12) kürzer als diejenigen (11) ohne solche Ansätze sind.

9. Filter nach einem der Ansprüche 1 bis 7, dadurch gekennzeichnet, daß die mit Ansätzen versehenen Klauen länger als diejenigen ohne solche Ansätze sind.

10. Filter nach einem der Ansprüche 1 bis 7, dadurch gekennzeichnet, daß die mit Ansätzen versehenen Klauen die gleiche Länge wie diejenigen ohne solche Ansätze aufweisen.

## Claims

1. A filter adapted to be placed in the course of blood flow, particularly in a venal flow to retain blood clots, of the type constituted by flexible lugs (11, 12) deployed substantially according to a conical corolla and emanating from an ogival or lozenge-shaped head (13), characterised in that at least some of the aforesaid lugs (12) are, towards their free ends, provided with appendices (14) orientated substantially parallel with the substantially cylindrical wall (16) generated by a line parallel with the axis (17) of the said conical corolla and describing like a directrix the perimeter of the opening (15) in the said corolla in a normal position of use.

2. A filter according to Claim 1, characterised in that the said appendices (14) extend at least partly from the free ends of the said lugs (12) which are provided thereon, being turned back substantially rearwardly and being directed towards the closure side of the cone.

3. A filter according to Claim 1 or Claim 2, characterised in that it comprises at least three lugs (12) provided with aforesaid appendices (14) distributed angularly about the aforesaid axis (17) of the filter.

4. A filter according to one of the preceding Claims, characterised in that it comprises at least three lugs (11) which do not have the aforesaid appendices (14) and which are distributed angularly about the aforesaid axis (17) of the filter.

5. A filter according to one of the preceding Claims, characterised in that the said appendices (14) are provided with means (24) for attachment to the vein.

6. A filter according to one of the preceding Claims, characterised in that the said appendices (14) are formed by appropriately folding over the ends of the corresponding lugs (12).

7. A filter according to one of Claims 1 to 5, characterised in that the said appendices (14) are folded back over the aforesaid lugs (12).

8. A filter according to one of the preceding Claims, characterised in that the lugs (12) which are provided with appendices (14) are shorter than those (11) which do not have such appendices.

9. A filter according to one of Claims 1 to 7, characterised in that the lugs which are provided with appendices are longer than those which do not have such appendices.

10. A filter according to one of Claims 1 to 7, characterised in that the lugs which have appendices are the same length as those which do not.

FIG_1

FIG_2

FIG_3

FIG_4

FIG_5

FIG_6

FIG_7

FIG_8

FIG_9

FIG_10

FIG_11

FIG_12